# EUROPÄISCHE PATENTSCHRIFT

(11) **EP 1 579 130 B1**
(45) Veröffentlichungstag und Bekanntmachung des Hinweises auf die Patenterteilung: **06.03.2013**
(21) Anmeldenummer: 03789097.7
(22) Anmeldetag: 27.11.2003
(51) Int. Cl.: A61M 5/145, F04B 19/00, F04B 53/16, A61M 5/142, A61M 37/00, F16J 15/32, F04B 53/14

(54) **KOLBENPUMPSYSTEM**
PISTON PUMP SYSTEM
SYSTEME POMPE A PISTON

(30) Priorität: 06.12.2002 EP 02027243
(43) Veröffentlichungstag der Anmeldung: 28.09.2005
(73) Patentinhaber: Boehringer Ingelheim International GmbH, 55218 Ingelheim am Rhein (DE)
(72) Erfinder: GESER, Johannes, 55218 Ingelheim (DE); EIGEMANN, Jutta, 44139 Dortmund (DE)
(86) Internationale Anmeldenummer: PCT/EP2003/013368
(87) Internationale Veröffentlichungsnummer: WO 2004/053362

(56) Entgegenhaltungen:
- EP-A- 0 361 260
- DE-A- 19 921 951
- US-A- 4 246 833
- US-A- 6 132 755
- US-B1- 6 389 955

## Beschreibung

Die vorliegende Erfindung betrifft ein Kolbenpumpsystem zum im Wesentlichen gasfreien Abmessen und/oder Umpumpen vorbestimmter Mengen an Flüssigkeiten, bevorzugt pharmazeutischen Flüssigkeiten mit oxidationsempfindlichen Bestandteilen. Bevorzugt wird das System als Mini- oder Mikropumpe oder Bestandteil einer solchen in Medizingeräten wie beispielsweise transdermalen therapeutischen Systemen eingesetzt.

### Stand der Technik

In Medizingeräten zum Ausbringen von pharmazeutischen Flüssigkeiten ist es oft notwendig definierte Volumina der Flüssigkeit mittels eines Pumpsystems aus einem Vorratssystem abzumessen und zum Ort des Ausbringens zu überführen. Je kleiner und handlicher das Medizingerät ist, um so kleiner muss die Pumpe beschaffen sein. Bei herkömmlichen Abfüllsystemen und/oder Pump-betriebenen Transportsystemen kann es vorkommen, dass die Flüssigkeit während der Überführung von einem Raum in einen anderen mittels der Pumpe mit dem Gas aus der Umgebung in Kontakt kommt oder sich dieses Gas mit der Flüssigkeit vermischt. Ein solches Durchdringen von Gas und Flüssigkeit ist nicht immer erwünscht. Dieser Effekt ist in verschiedenen Fällen nicht tolerabel. So gibt es z.B. Flüssigkeiten mit oxidationsempfindlichen Substanzen und das Vermischen mit Sauerstoff z.B. aus der Luft beeinträchtigt maßgeblich die pharmazeutische Qualität des Formulierung. In anderen Fällen kann das so in die Abmessskammer eingedrungene Gas den Abmessvorgang verfälschen und damit die auszubringende Menge der Flüssigkeit verändern. In anderen Fällen, bei denen die Flüssigkeit parenteral appliziert wird, z.B. intravenös, darf die auszubringende Flüssigkeit per se keine größeren Mengen an Gas enthalten, um die Gesundheit des Patienten nicht zu gefährden.

Dieses Problem der Gaseindringung tritt insbesondere bei Kolbenpumpsystemen auf, bei denen die abzufüllende Flüssigkeit aus einem Vorratssystem über einen sich hubartig hin- und herbewegende Kolben in eine Abmesskammer überführt wird und von dort aus dem weiteren Bestimmungsort zugeführt wird. In solchen Systemen bewegt sich der Pumpkolben in einer Führungsröhre und ist gegenüber dieser abgedichtet. Die Dichtung soll dann verhindern, dass es zu einem Austreten der Flüssigkeit aus dem Abfüllraum oder zu einem Eindringen von Gas in den Abfüllraum kommen kann.

Die WO 01/89612 A1 beschreibt ein Kolbenpumpensystem zum Ausbringen von kleinen Flüssigkeitsmengen von 0,01 ml bis 2 ml, bei welchem ein in einem Zylinder beweglicher Kolben über eine Verbrennung wie bei einem Verbrennungsmotor bewegt wird und dabei eine kleine Wirkstoffmenge über einen angelagerten Zapfen, der mit einer Kammer in Verbindung steht, austreibt.

Die US 4,246,833 offenbart ein Kolbenpumpensystem für Hochdruckanwendungen, bei dem eine O-Ringdichtung aus beispielsweise Silikon ein dichtendes Fitting des Kolbens zu einem Zylinder bewerkstelligt.

Die EP 0361260 A2 offenbart ein Kolbenpumpensystem mit einem Mechanismus zum Verändern eines Nachtropfens, bei dem der Zylinder mittig eingeformt ist, um einen Ring zu bilden, auf dem eine O-Ringdichtung aufliegt.

Die DE 19921951 A1 offenbart eine Piezo-Kolbenpumpe, wobei der Kolben sowohl eine Umfangs-Ringdichtung zum Zylinder, als auch eine stirnseitige Ringdichtung zu einem Druckraum aufweist.

Bei der WO 01/89612 A1, der US 4,246,833, der EP 0361260 A2 und der DE 19921951 A1 sind die O-Ringdichtungen analog zu allgemein bekannten Kolbenringen in Nuten in oder auf der Außenfläche des Kolbens angeordnet. Die Nuten sind dabei umlaufende Ausnehmungen in der Kolbenaußenfläche.

Für die Erfindung geeignete Medizingeräte werden z.B. transdermale therapeutische Systeme wie sie in der EP 0840634 offenbart. Solche Systeme bestehen im Wesentlichen aus einem Reservoir für das Arzneimittel und mindestens einem - typischerweise mehreren - mit kapillaren Öffnungen versehenen Mikrostacheln, die so mit dem Reservoir in Verbindung srehen, daß das Arzneimittel in Form einer wirkstoffhaltigen Lösung aus dem Reservoir in die Mikrostacheln gelangt. Bei Aufsetzen des transcornealen Systems auf die Haut wird das Stratum corneum und gegebenenfalls die Epidermis von den Mikrostacheln durchdrungen, so daß ein direkter Zugang zur innervierten Hautschicht gegeben ist. Damit kann das Arzneimittel aus dem Reservoir durch die kapillaren Öffnungen der Mikrostacheln bis in vaskularisierte Abschnitte der Haut gelangen, um dort über das kapillare Kreislaufsystem in den Blutkreislauf aufgenommen zu werden. In den Systemen liegt der Wirkstoff üblicherweise in Form einer Lösung vor, um einen einwandfreien Transport durch die kapillaren Öffnungen der Mikrostacheln des transcornealen Systems zu gewährleisten.

Der Transport des Medikaments kann "aktiv", beispielsweise durch einen im Reservoir gespeicherten Überdruck, elektrostatische oder kapillare Kräfte, oder eine im System integrierte Pumpe erfolgen. Ein solches aktives System wird in der EP 0840634B1 beschreiben.

### Beschreibung der Erfindung

Es ist Aufgabe der vorliegenden Erfindung ein kolbenbetriebenes Pumpsystem bereit zu stellen, welches gewährleistet, dass beim Abmessen und/oder Umpumpen von Flüssigkeiten mittels eines Kolbenpumpsystems im wesentlichen keine Gas entlang dem Kolben von Außen in die Abfüll- oder Pumpkammer gelangen kann.

Eine weitere Aufgabe besteht darin, ein Pumpsystem für Medizingeräte zu schaffen, welches verhindert, dass durch das Abmessen oder Umpumpen die pharmazeutische Flüssigkeit mit Sauerstoff, Luft oder einem anderen Gas vermischt werden.

Eine weitere Aufgabe besteht darin, die aus dem Stand der Technik bekannten Nachteile von Pumpsystem in Medizingeräten zu überwinden.

### Detaillierte Beschreibung der Erfindung

Die vorliegende Erfindung betrifft nun ein Kolbenpumpsystem, das als oder in einer kleinen Pumpe, z.B. einer Mini- oder Mikropumpe, in Medizingeräten zu direkten Applikation von pharmazeutischen Formulierungen verwendet werden kann. Bevorzugt wird die erfindungsgemäße Vorrichtung in Medizingeräten, die zum Ausbringen von Flüssigkeiten eine Pumpe benötigen oder benötigen können, verwendet. Die Erfindung kann jedoch in jedem anderen nicht zwingend nur auf Medizingeräte beschränkten Kolbenpumpsystem angewendet werden, in dem sie vorteilhaft eingesetzt werden kann. Die Erfindung ist auch nicht auf Minipumpen oder Mikropumpen beschränkt, sondern kann auch auf größere Pumpsystem übertragen werden.

Im Rahmen der vorliegenden Erfindung werden bevorzugt unter dem Begriff Medizingerät, Applikationsapparaturen für Flüssigkeiten wie transdermalen therapeutische Systeme mit aktivem Wirkstofftransport, Geräte für die intravenöse Verabreichungen von flüssigen Formulierungen in Kleinstmengen, Zerstäuber von Flüssigkeiten, wie Inhalatoren, insbesondere treibgasfreie Inhalatoren, nadellose Injektoren, Augensprays etc. verstanden. Solche Medizingeräte dienen zum Teil auch als Primärpackmittel für pharmazeutische Zubereitungen oder können als solche betrachtet werden, da die pharmazeutische Zubereitung in diesen Geräten zunächst gelagert wird, bevor das Gerät am oder für den Patienten zum Einsatz kommt. Daher umfasst der Begriff ebenfalls Medizingeräte, die als Primärpackmittel dienen.

Erfindungsgemäß wird ein Kolbenpumpsystem bereitgestellt, welches unter Verwendung von Lebensmittel- oder Arzneimittel-technisch geeigneten Dichtungsmaterialien die Abdichtung des Kolbens im Pumpsystem gegenüber dem Eindiffundieren von Luft oder anderen Gasen aus der Außenumgebung in die abzufüllende oder abzumessende Flüssigkeit verbessert und damit das Eindringen von Luft oder anderen Gasen in diese Flüssigkeit reduziert. Durch das erfindungsgemäße Pumpsystem werden die oben genannten Nachteile der gängigen Pumpsysteme überwunden.

Ein für die Erfindung geeignetes Pump- und Abmesssystem kann aus einer Kammer bestehen, die mit einem Flüssigkeitseinlass und einem Flüssigkeitsauslass versehen ist, wobei ein Kolben so mit der Kammer verbunden ist, dass durch entlang seiner Längsachse hubartige Bewegung des Kolbens Flüssigkeit über den Flüssigkeitseinlass aus einem Vorratssystem in einer vorbestimmten Menge angesaugt werden kann und dann von dort aus über den Flüssigkeitsauslass gegebenenfalls unter Druck ausgebracht wird.

Zwischen zwei Hubbewegungen kann das System ruhen. In manchen Fällen ist das Vorratssystem als flexibler Behälter ausgebildet, der mit der Entnahme von Flüssigkeit zusammenfällt. In solchen Vorratssystems, insbesondere, Systemen mit sehr kleinen Abmessungen, d.h. eine Abmess- oder Pumpkammer mit einem Volumen im Mikroliter-Bereich und entsprechenden Querschnitten der Zu- und Abführwege und einem Vorratssystem, kann es dann in der Kammer in der Ruhephase zu einem statischen Unterdruck bzw. statischen Vakuum kommen. D.h. der Unterdruck im Vorratssystem wird an die Pumpkammer weitergegeben.

Das erfindungsgemäße Pumpsystem ist dementsprechend sowohl für eine dynamische Hochdruckbelastung ausgelegt, als auch für eine dynamische und/oder statische Unterdruckbelastung. Unter Hochdruck werden Drücke von mehr als 1 bar verstanden. Bevorzugt ist das System auf einen Druck von bis zu 600 bar ausgelegt, besonders bevorzugt bis zu 250 bar. Dieser Druck kann bis zu ca. 10 Sekunden, bevorzugt bis zu 5 Sekunden, stärker bevorzugt bis zu 2 Sekunden aufrecht erhalten werden.

Unter Unterdruck wird eine Druckdifferenz von bevorzugt weniger als 0,5 bar, bevorzugt weniger als 100 mbar und stärker bevorzugt von weniger als 50 mbar verstanden. Unter einem statischen Unterdruck wird verstanden, dass dieser über einen Zeitraum von mehr als 5 Minuten, bevorzugt mehr als 1 Stunde, bevorzugt mehr als 10 Stunden und am stärksten bevorzugt von ca. 24 Stunden aufrecht erhalten wird.

Im Rahmen der vorliegenden Beschreibung wird die oben erwähnte Kammer auch als Pumpkammer oder Abmesskammer bezeichnet. Bevorzugt weist die Kammer ein Füllvolumen von 1 Mikroliter bis zu 1 ml auf, stärker bevorzugt 1 Mikroliter bis zu 500 Mikrolitern, insbesondere bevorzugt von 5 Mikrolitern bis zu 100 Mikrolitern. Am stärksten bevorzugt sind Volumina von 5 Mikrolitern bis zu 30 Mikrolitern.

Der Flüssigkeitseinlass bzw. das mit dem Flüssigkeitseinlass verbundene Zuliefersystem, welches die Flüssigkeit aus dem Vorratssystem in die Kammer führt, wird bevorzugt von Röhren oder Schläuchen gebildet. Der Querschnitt der Rohröffnung beträgt bevorzugt weniger als 1 mm, stärker bevorzugt weniger als 0,5 mm.

Das Zuliefersystem weist bevorzugt ein Rückschlagventil auf, welches ein Rücklaufen der angesaugten Flüssigkeit in den Vorratsbehälter verhindert. Das System aus Flüssigkeitseinlass und/oder das Zuliefersystem wird im Rahmen der vorliegenden Erfindungsbeschreibung auch als Ansaugsystem bezeichnet.

Das Ansaugsystem kann gegebenenfalls in dem Pumpkolben integriert werden. In diesem Fall handelt es sich bei dem Pumpkolben um einen Hohlkolben. Der hohle innere Bereich des Kolbens stellt dann den Zulauf für die Flüssigkeit aus dem Vorratssystem in die Kammer dar oder ist mit einem solchen Zulauf verbunden. In diesem Fall enthält das Ansaugsystem ein Rückschlagventil, bevorzugt als integraler Bestandteil des Kolbens.

Der Flüssigkeitsauslass, bzw. das mit dem Flüssigkeitsauslass verbundene Abführsystem für die Flüssigkeit, welches die Flüssigkeit aus der Pump- oder Messkammer seinem weiteren Bestimmungsort zuführt, kann - muss aber nicht - ein solches Rückschlagventil aufweisen. Der Flüssigkeitsauslass weist in jedem Fall dann ein Rückschlagventil auf, wenn das Abfüllsystem dergestalt ist, dass ein Rücklaufen der aus der Abmesskammer herausgepressten Flüssigkeit vom Bestimmungsort über den Flüssigkeitsauslass zurück in die Pump- bzw. Abmesskammer möglich oder unerwünscht ist. Vergleichbar ist die Situation, wenn Gefahr besteht, dass über das Abführsystem Luft von außen in die Pumpkammer gezogen werden kann. Auch dann ist ein Rückschlagventil sinnvoll.

Das System Flüssigkeitseinlass und/oder das besagte Abführsystem wird im Rahmen der vorliegenden Erfindungsbeschreibung auch als nur als Abführsystem bezeichnet.

Der in die Pump- bzw. Abmesskammer ragende bewegliche Kolben wird in einer zylindrischen Bohrung eines festen Elements geführt, beispielsweise einem Block oder einer Wand. Ein solcher Block oder eine solche Wand kann in dem Pumpsystem ein eigenständiges Element sein oder ist integraler Bestandteil der Kammer. Der Kolben kann über eine vorbestimmte Hubbewegung in die Kammer eingeführt werden oder aus dieser herausgezogen werden. Durch diese Hubbewegung wird die Kammer gefüllt und geleert. Die Dimensionen des Kolbens und der Kammer sollten entsprechend aufeinander abgestimmt sein.

Der Kolben weist bevorzugt eine Länge von 5 mm bis zu 10 cm auf, bevorzugt von 1 cm bis zu 7,5 cm.

Der Querschnitt des Kolbens beträgt bevorzugt 0,25 bis 4 mm, stärker bevorzugt 0,5 bis 3 mm und am stärksten bevorzugt 0,75 bis 2,25 mm.

Die Hubbewegung des Kolbens entlang seiner Längsachse überstreicht bevorzugt eine Länge von 1 mm bis zu 5 cm, insbesondere von 0,25 cm bis zu 3 cm. Stärker bevorzugt sind Hubbewegungen von 0,5 cm bis 2 cm.

Eine Dichtung am Kolben dichtet unabhängig von dessen Bewegung den Raum zwischen dem Koben und der Kammer ab und verhindert so, dass Flüssigkeit austreten kann. D.h. der Kolben wird in dem Pumpsystem so geführt, dass er im Normalbetrieb nicht aus der zylindrischen Führung ausbrechen kann und die seine Dichtungswirkung stets erfüllt.

An das Dichtungsmaterial - wie auch an alle anderen Materialien von Systemen zum Abfüllen von pharmazeutische Flüssigkeiten, sind besondere Anforderungen gestellt. So müssen sie derart beschaffen sein, dass weder die pharmazeutische Qualität der Flüssigkeit beeinträchtigt wird, noch das es zu Verunreinigungen kommt, die die Gesundheit des Endkonsumenten gefährden. Diese Anforderungen gelten insbesondere auch an das Dichtungsmaterial, das sich hierbei in der Regel um elastische Polymere handelt, aus denen während der Verwendung immer wieder Bestandteile austreten können, die in den abzufüllenden Flüssigkeiten aus den oben genannten Gründen unerwünscht sind. In diesem Zusammenhang wird auf die öffentliche Diskussion um Weichmacher etc. in Verpackungsmaterialien für Nahrungsmittel u.ä. hingewiesen.

Daher müssen im Rahmen der erfindungsgemäßen Pumpe die Dichtungssysteme für den Hubkolben primär so ausgesucht werden, dass sie die Qualität der Flüssigkeit nicht verändern können. Diesem Kriterium müssen sich die funktionsbedingten Rahmenbedingungen unterordnen. Zu solchen sekundären Eigenschaften des Dichtungsmaterials gehören die Dichte des Materials und/oder deren Permeationskoeffizienten für Luft oder andere Gase. Erfindungsgemäß wird die Kolbenführung in dem Pumpsystem bevorzugt über Silikon-Dichtungen geführt, da Silikon die genannten lebensmittel-geeigneten bzw. pharmazeutisch akzeptablen Eigenschaften aufweist. Ein Nachteil dieses Dichtungsmaterials und bezüglich der Lebensmittel- oder Arzneimittel-gesetzlichen Bestimmungen geeigneten anderer Dichtungsmaterialien besteht allerdings darin, dass diese Materialien einen relativ hohen Permeationskoeffizienten für Luft aufweisen, so dass durch das Dichtungsmaterial Luft und Sauerstoff in die abzufüllende Flüssigkeit diffundieren kann. Gängige Dichtungsmaterialien wie zum Beispiel bei NBR oder PU können aus pharmazeutischen Gründen nicht in allen Fällen eingesetzt werden.

Bevorzugt ist die Dichtung als O-Ringdichtung ausgeführt. Unter O-Ring wird eine ringförmige Dichtung verstanden, unabhängig von der Form ihres Querschnitts. Bevorzugt sind O-Ringdichtungen mit kreisrundem Querschnitt.

Der Kolben kann durch einen oder mehrere O-Ringe abgedichtet werden. Bevorzugt befindet sich wenigstens eine Dichtung in der Führungsröhre des Kolbens in der Nähe des Eintrittsorts des Kolbens in die Pumpkammer.

Der Stand der Technik sieht beim Einbau von derartigen O-Ringen zur Abdichtung der dynamisch belasteten Kolbenstange eine Querschnittsverpressungen von 10 % bis ca. 15 % vor. Dadurch sei ein ausgeglichenes Verhältnis von Verschleiß und Dichtheit gegeben.

Es hat sich nun überraschend gezeigt, dass diese Einbauverhältnis in den erfindungsgemäßen Medizingeräten nicht zu einer ausreichenden Dichtigkeit für die statische Vakuumabdichtung führt.

Für solche Abdichtungen werden Verpressungen von über 50 % und einem Nutfüllgrad von üblicherweise 80%, bei Vakuumabdichtung von bis zu 100% gelehrt (Wilhelm Schmitt, "Kunststoffe und Elastomere in der Dichtungstechnik", W.Kohlhammer GmbH 1987, Kapitel 2.1.4, Seite 228, Kapitel 2.2.1, Seite 231). Im Fall von statischen Vakuumdichtungen gelten nach dem zitierten Stand der Technik Verpressungsgrade von größer 50%, die Verwendung von Vakuumfett, eine Rauhtiefe von Nut und Gegenstelen von kleiner als 0,5 Mikrometer und eine sehr geringe Gaspermeabilität als zwingende Voraussetzungen. Um die geringe Gaspermeabilität zu gewährleisten sind folgende O-Ring-Basiselastomere bekannt: Butylkautschuk, Epichlorhydrin, Fluorcarbonkautschuk und Nitilkautschuk mit hohem Acrylnitirilgehalt.

Als gänzlich ungeeignet werden Silikonkautschuke betrachtet, da sie besonders hohe Gaspermeabilitäten aufweisen.

Die folgende Tabelle gibt einen Überblick über die Gaspermeabilitäten verschiedener Stoffe. Die Gaspermeation wird über den Gaspermeationskoeffizienten P verglichen [N*cm³*mm/(m²*h*bar)] für Stickstoff (N₂):

| | P bei 20°C | P bei 80°C |
|---|---|---|
| Butyl | 0,2 - 0,6 | 8-15 |
| Epichlorhydrin Copolymer | 0,5 - 4 | 10-40 |
| Epichlorhydrin Homopolymer | 0,04 | 1 - 5 |
| Fluorcarbon | 0,2 - 2 | 12 - 4ß |
| Nitril | 0,5 - 5 | 15 - 100 |
| Ethylen-Propylen | 2 - 10 | 40 - 100 |
| Polytetrafluorethylen | 1,5 - 5 | 5 - 30 |
| Polyurethan | 0,5 - 1,3 | 18 - 50 |
| | | |
| Silicon | 100 - 500 | 500 - 1200 |

Derartig hohe Verpressungen führen aber zu nicht akzeptablen Verschleiß und zu sehr hohen Reibungskräften bei dynamischer Belastung. Der Verscheiß gefährdet auf Dauer nicht nur das Einhalten der erfindungsgemäßen Aufgabe, sondern kann auch zu einer Belastung der Flüssigkeit in der Pumpkammer und damit der Wirkstoffformulierung mit abgeriebenen Partikeln führen, was aus pharmazeutischer Sicht nicht akzeptabel ist.

Überraschend wurde nun gefunden, dass entgegen den Annahmen aus dem Stand der Technik das Problem der nicht ausreichend permeations- bzw. diffusionsdichten Abdichtung der O-Ringdichtung bei statischen Vakuumabdichtungen mit Dichtungen, die einen Gaspermeationskoeffizienten von 100 bis 500 N*cm³*mm/(m²*h*bar) aufweisen, dadurch gelöst werden kann, dass weder die Verpressung der Dichtung erhöht wird, noch dass die Dichtung gefettet werden muss, sondern dass allein der Ausfüllgrad der Dichtung in der sie haltenden Nut (Nutfüllgrad) optimal eingestellt wird.

Erfindungsgemäß wird die O-Ringdichtung mit einer radialen und ggf. axialen Verpressung von bis zu 30%, bevorzugt bis zu 20% geführt. Unter radialer Verpressung wird die Verpressung verstanden die entlang der Ringebene auf den Dichtungsring ausgeübt wird.

Erfindungsgemäß wird nun eine O-Ringdichtung mit einer Gaspermeation von 100 bis 500 N*cm³*mm/(m²*h*bar) und einer Schnurstärke von 0,3 bis 3 mm, bevorzugt 0,5 bis 2 mm, stärker bevorzugt 0,75 bis 1,5 mm vorgeschlagen, die einen sich entlang seiner Längsachse hin- und herbewegenden Hubkolben in einer Führungsröhre abdichtet und in einer Nut gehalten wird, wobei die Dichtung eine radiale und ggf. axiale Verpressung von bis zu 30%, bevorzugt bis zu 20% aufweist und einen Nutfüllgrad von 90 bis 100% aufweist.

Unter einem Nutfüllgrand von 90% wir verstanden, dass das 90% des Volumens der Nut mit der Dichtung gefüllt sind.

Bevorzugtes Dichtungsmaterial ist Silicon.

Die erfindungsgemäße Pumpe kann mechanisch oder elektrisch betrieben werden. Einzelheiten können dem Stand der Technik entnommen werden. Diese Ausführungsformen können elektronisch gesteuert sein, bevorzugt über einen Mikrochip.

Der Kolben kann z.B. durch Koppelung mit einem piezoelektrischen Element betrieben werden. Diese Koppelung kann direkt sein, über einen oder mehrere Hebelarme oder eine Membran. Bevorzugt wird der Kolben direkt von dem piezoelektrischen Element bewegt. Das piezoelektrischen Element selbst wird in einem solchen Fall z.B. über den Mikrochip angesteuert.

Der Kolben kann auch über eine Feder, z.B. Schraubenfeder, betrieben werden, die mechanisch oder elektrisch gespannt wird und mit dem Kolben über einen Flansch verbunden ist. Einzelheiten können dem Stand der Technik für Medizingeräte, insbesondere aus den Bereichen transdermale therapeutische Systeme, Zerstäuber, treibgasfreie Inhalatoren, nadellose Injektoren etc. entnommen werden.

Prinzipiell können mit den erfindungsgemäßen Medizingeräten und dem beschriebenen Pumpsystem alle physiologisch verträglichen Lösungsmittel oder Lösungsmittelgemische, in denen sich der Wirkstoff in ausreichender Menge löst, verwendet werden. Unter einer ausreichenden Menge werden solche Konzentrationen an Wirkstoff im Lösungsmittel verstanden, die es ermöglicht, eine therapeutisch wirksame Menge Wirkstoff applizieren zu können.

Bevorzugte Lösungsmittel sind Wasser und pharmakologisch verträgliche Alkohole, wie Ethanol. Sollte es notwendig sein, können zur Erhöhung der Löslichkeit des Wirkstoffes im Lösungsmittel Lösungsvermittler und Komplexbildner verwendet werden. Empfindliche Wirkstoffe können zur Erhöhung der Lagerstabilität mit Zusätzen versehen werden.

Das erfindungsgemäße Medizingerät enthält ein Reservoir zur Speicherung der Wirkstofflösung, eine flüssigkeitsleitende Verbindung zwischen dem Reservoir und der erfindungsgemäßen Pumpe, und eine flüssigkeitsleitende Verbindung zu wenigstens einer die Flüssigkeit ausbringenden Einrichtung. Letztere kann eine Düse, ein Mikrostachel, eine Mikroschneide sein, an der die Flüssigkeit entlang geführt wird, eine Kanüle oder ein Auslauf. Mikroschneiden und Mikrostacheln werden ausführlich in der EP 0840634 und dort in den Figuren 6 beschrieben, Düsensysteme können der EP 1017469 entnommen werden. Solche Düsensysteme können eine einzige Düsenöffnung enthalten oder mehrere Düsenöffnungen. Eine solche Düse kann ein Körper sein mit wenigstens zwei oder mehr durchgehenden Bohrungen, die parallel zueinander verlaufen oder gegeneinander geneigt sind. Im Fall von gegeneinander geneigten Bohrungen, bildet die Seite mit dem spitzen Winkel die Düsenauslaßseite, die andere Seite entsprechend die Düseneinlaßseite.

Mit den erfindungsgemäßen Geräten werden bevorzugt geringe Volumina an Flüssigkeiten, z.B. weniger als 1 ml oder sogar weniger als 100 Mikrolitern, aus einem Vorratssystem über das Pump- und/oder Abmesssystem in eine Pumpkammer hinein abgemessen und von dort aus zu der die Flüssigkeit ausbringenden Einrichtung geführt . Solche Systeme umfassen z.B. transcomeale therapeutische Systeme (TTS, "Pflaster"-Systeme), Zerstäuber, insbesondere Pumpsysteme in Nasensprays, Inhalatoren, Augenduschen, Infusionssystem, nadellose Injektoren etc., vorausgesetzt, dass sie treibmittelfreie Flüssigkeiten abmessen und ausbringen, um sie einem Patienten zuzuführen.

Transcorneale therapeutische Systeme überführen kontinuierlich oder diskontinuierlich pharmazeutische Formulierungen aus einem Vorratsbehälter über die Haut in einen Patienten. So kann das erfindungsgemäße Pumpsystem z.B. in ein TTS wie es von der EP 0840634, auf die hiermit ausdrücklich Bezug genommen wird, beschrieben ist, eingebaut werden. Ein solches System kann aus einem Vorratssystem bestehen, in dieses ragt der Pumpkolben des erfindungsgemäßen Pumpsystems, welcher bevorzugt als Hohlkolben mit integriertem Rückschlagventil ausgebildet ist. Der Hohlkolben mündet in die Abmesskammer von der aus ein Abführsystem in einen oder mehre stachelförmige Fortsätze führt. Dieser oder diese stachelförmige(n) Fortsatz (Fortsätze) sind ebenfalls hohl und so ausgebildet, dass sie - wenn das Pflastersystem gebrauchsfertig mit der Haut des Patienten verbunden ist - in das Corneum des Patienten einstechen und dort die Flüssigkeit hineingepumpt werden kann. In einem solchen System weist das Abführsystem, welches aus wenigsten einem Rohr besteht bevorzugt ein Rückschlagventil (oder mehrere Rückschlagventile) auf.

### Beschreibung der Figuren

Bei einer Kolbenpumpe zur Dosierung von sehr kleinen Volumina ist, bei einem einzelnen Kolbenhub, eine Flüssigkeitsmenge von ca. 15 *µ*L sehr präzise zu fördern. Dies muss auch bei der ersten Betätigung nach längerer Nichtbenutzung der Fall sein. Um dies sicherzustellen, darf während der Nichtbenutzung keine Luft in die Pumpe eintreten, da sonst die Dosierung nicht mehr in der gewünschten Genauigkeit erfolgen kann.

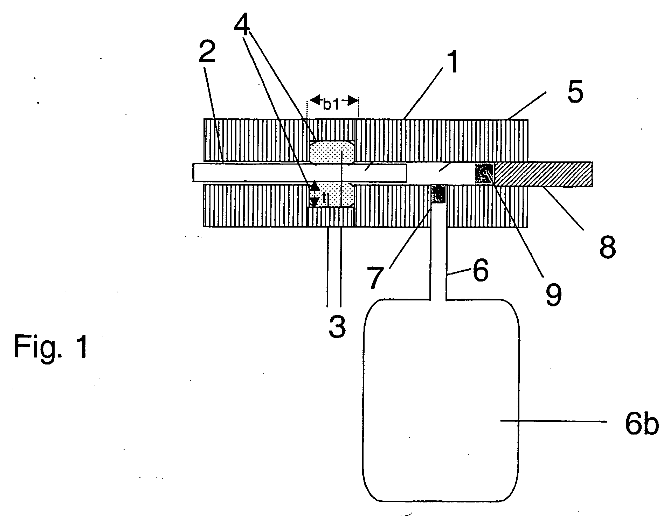

Figur 1 zeigt ein solches Pumpsystem mit einem Kolben 1 von 1,5 mm Durchmesser. Der Kolben mündet in der Pumpkammer 5. Der Kolben wird in einer Führungsröhre 2 geführt und über eine O-Ringdichtung 3 in einer Nut 4 abgedichtet. Der O-Ring weist zum Beispiel eine Schnurstärke von dl = 1,1 mm auf und es wird eine radiale Verpressung von ca. 20 % eingestellt. Dadurch ergibt sich eine Nuttiefe von t = 0,9 mm. Zum Einstellen des Nutfüllgrads wird eine Nutbreite b₁ = 1,1 mm gewählt, so dass ein Nutfüllgrad von 95 % eingestellt wird.

Aus chemischen Gründen wird ein Silikonelastomer als Dichtungswerkstoff eingesetzt.

Der Kolben führt in die Pump- und Abmesskammer 5. In die Kammer mündet eine Zufuhrröhre 6 mit einem Rückschlagventil 7, durch welche Flüssigkeit angesaugt wird.

Aus der Kammer führt eine Abführröhre 8 mit dem Rückschlagventil 9 Flüssigkeit ab. Die Vorratskammer trägt das Bezugszeichen 6b.

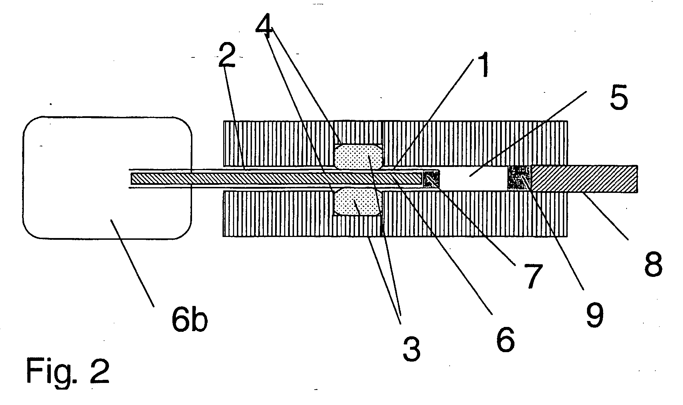

Figur 2 zeigt das System nach Figur 1, bei welchem die Zuführöhre 6 in dem Kolben 1 integriert ist.

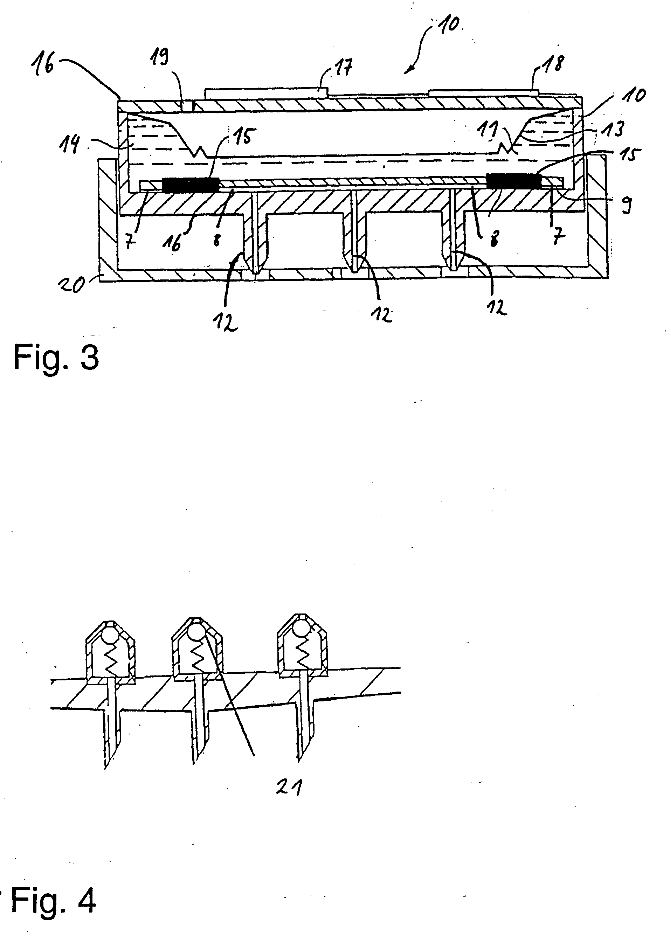

Figur 3 zeigt ein transdermales therapeutisches System mit der erfindungsgemäßen Pumpe. Die Figur zeigt einen Schnitt durch ein transcomeales System 10 mit einem Wirkstoffreservoir 11, das an seiner Oberseite durch einen Faltenbalg 13 abgeschlossen ist. In dem Wirkstoffreservoir befindet sich die Wirkstofflösung 14, die an der unteren Seite des Wirkstoffreservoirs über ein System 15 nach Figur 2 zu den an der Unterseite des Gehäuses angeordnete Mikrostacheln mit kapillaren Öffnungen 12 nach außen befördert wird. Die Gehäuseseitenteile 16 und die Gehäuseunterseite bilden zusammen mit den Mikrostacheln eine konstruktive Einheit, bevorzugt aus einen thermoplastischen Kunststoff. Der Deckel des Gehäuses enthält die Energieversorgung in Form einer Batterie 17 zum Betreiben des Pumpsystems sowie eine elektronische Steuerung 18, z.B. einen Mikrochip. Eine Belüftung 19 ermöglicht, daß der Faltenbalg bei der Abgabe von Wirkstofflösung durch die Mikrostacheln sich diesem verringerten Volumen anpassen kann. Vor dem Gebrauch des transcornealen Systems sind die Mikrostacheln durch einen Stachelschutz 20, beispielsweise in Form einer Kappe, geschützt.

Gegebenenfalls können die Mikrostacheln Mikroventile 21 beinhalten, wie sie Figur 4 dargestellt sind.

## Patentansprüche

1. Kolbenpumpsystem zur Dosierung pharmazeutischer Flüssigkeiten mit Pumpvolumen von 1 Mikroliter bis 1 ml bestehend aus einem in einer Führungsröhre (2) geführten Kolben (1), der entlang seiner Längsachse eine Hubbewegung ausführen kann, der in eine Pumpkammer (5) mündet, wobei die Pumpkammer (5) über eine flüssigkeitsleitende Verbindung mit Ventil (7) mit einem Vorratsgefäß (11) verbunden ist und von der Pumpkammer (5) eine flüssigkeitsleitende Verbindung zu einer Einrichtung (8) zum Ausbringen der Flüssigkeit führt, **dadurch gekennzeichnet, dass** die Führungsröhre (2) eine Nut (4) umfasst, in der eine O-Ringdichtung (3) angeordnet ist, die den Kolben (1) abdichtet und wobei die Dichtung einen Gaspermeationskoeffizienten von 100 bis 500 N*cm³*mm/(m²*h*bar) für Stickstoff (N₂), eine radiale Verpressung von weniger als 30% aufweist und die Dichtung (3) die Nut (4) mit einem Nutfüllgrad von mehr als 90% füllt und wobei der Kolben (1) einen Querschnitt von 0,75 bis 2,25 mm aufweist.

2. Kolbenpumpsystem nach Anspruch 1, **dadurch gekennzeichnet, dass** der Nutfüllgrad mehr als 95% beträgt.

3. Kolbenpumpsystem nach Anspruch 1 oder 2, **dadurch gekennzeichnet, dass** das Ventil ein Rückschlagventil (7) ist.

4. Kolbenpumpsystem nach einem der Ansprüche 1 bis 3, **dadurch gekennzeichnet, dass** in der Verbindung zu einer Einrichtung zum Ausbringen der Flüssigkeit ein Rückschlagventil (9) ausgebildet ist.

5. Kolbenpumpsystem nach einem der Ansprüche 1 bis 4, **dadurch gekennzeichnet, dass** der Kolben (1) eine Länge von 5 mm bis zu 10 cm aufweist, bevorzugt von 1 cm bis zu 7,5 cm.

6. Kolbenpumpsystem nach einem der Ansprüche 1 bis 5, **dadurch gekennzeichnet, dass** die Hubbewegung des Kolbens (1) entlang seiner Längsachse eine Länge von bis zu 1 mm bis zu 5 cm, bevorzugt von 0,25 cm bis zu 3 cm, stärker bevorzugt von 0,5 cm bis 2 cm überstreicht.

7. Kolbenpumpsystem nach einem der Ansprüche 1 bis 6, **dadurch gekennzeichnet, dass** die O-Ringdichtung (3) aus Silikon besteht.

8. Kolbenpumpsystem nach einem der Ansprüche 1 bis 7, **dadurch gekennzeichnet, dass** der Kolben (1) ein Hohlkolben ist, in dem die flüssigkeitsleitende Verbindung mit Ventil (7), welche die Pumpkammer (5) mit einem Vorratsgefäß (11) verbindet, integriert ist.

9. Kolbenpumpsystem nach einem der Ansprüche 1 bis 8, **dadurch gekennzeichnet, dass** die Bewegung des Kolben (1) mechanisch gesteuert wird.

10. Kolbenpumpsystem nach Anspruch 9, **dadurch gekennzeichnet, dass** der Kolben (1) über eine Schraubenfeder bewegt wird.

11. Kolbenpumpsystem nach einem der Ansprüche 1 bis 8, **dadurch gekennzeichnet, dass** die Bewegung des Kolben elektronisch gesteuert wird.

12. Kolbenpumpsystem nach Anspruch 11, **dadurch gekennzeichnet, dass** der Kolben mittels eines Mikrochip (18) gesteuert wird.

13. Kolbenpumpsystem nach Anspruch 11 oder 12, **dadurch gekennzeichnet, dass** der Kolben mittels eines piezoelektrischen Elements bewegt wird.

14. Kolbenpumpsystem nach einem der vorangehenden Ansprüche, **dadurch gekennzeichnet, dass** die Einrichtung zum Ausbringen der Flüssigkeit wenigstens eine Düse, wenigstens ein Mikrostachel, wenigstens eine Mikroschneide sein, an der die Flüssigkeit entlang geführt wird, wenigstens Kanülen und/oder wenigstens ein Auslauf ist.

15. Kolbenpumpsystem nach einem der vorangehenden Ansprüche, **dadurch gekennzeichnet, dass** die Schnurstärke des O-Rings (3) 0,3 bis 3 mm beträgt, bevorzugt 0,5 bis 2 mm, stärker bevorzugt 0,75 bis 1,5 min.

16. Medizingerät zum Ausbringen von pharmazeutischen Flüssigkeiten beinhaltend ein Kolbenpumpsystem nach einem der Ansprüche 1 bis 15.

17. Medizingerät nach Anspruch 16, **dadurch gekennzeichnet, dass** das Medizingerät ein transdermal, therapeutisches System ist, welches neben dem Kolbenpumpsystem ein Vonatsgefäß aus wenigstens einem beweglichen Element oder mit einer Entlüftungsöffnung aufweist und wenigstens eine Mikrostachel oder Mikroschneide.

18. Medizingerät nach Anspruch 16, **dadurch gekennzeichnet, dass** das Medizingerät ein Zerstäuber für Flüssigkeiten, ein Nasenspray, ein Augenspray, ein Inhalator, bevorzugt ein treibgasfreier Inhalator, ein nadelloser Injektor oder ein Infusionsgerät ist.

## Claims

1. Piston pump system for metering pharmaceutical liquids, with a pumping volume of 1 microlitre to 1 ml, consisting of a piston (1) guided within a guide tube (2) and capable of performing a stroke movement along its longitudinal axis, opening into a pumping chamber (5), the pumping chamber (5) being connected via a liquid-conveying connection with valve (7) to a storage vessel (11) and from the pumping chamber (5) a liquid conveying connection leads to a device (8) for delivering the liquid, **characterised in that** the guide tube (2) comprises a groove (4) in which is disposed an O-ring seal (3) which seals off the piston (1) and wherein the seal has a gas permeation coefficient of 100 to 500 N*cm³*mm/(m²*h*bar)] for nitrogen (N₂), a radial compression of less than 30% and the seal (3) fills the groove (4) with a groove filling level of more than 90%, and wherein the piston (1) has a cross-section of 0.75 to 2.25 mm.

2. Piston pump system according to claim 1, **characterised in that** the groove filling level is more than 95%.

3. Piston pump system according to claim 1 or 2, **characterised in that** the valve is a non-return valve (7).

4. Piston pump system according to one of claims 1 to 3, **characterised in that** a non-return valve (9) is formed in the connection to a device for delivering the liquid.

5. Piston pump system according to one of claims 1 to 4, **characterised in that** the piston (1) has a length of 5 mm to 10 cm, preferably from 1 cm to 7.5 cm.

6. Piston pump system according to one of claims 1 to 5, **characterised in that** the stroke movement of the piston (1) along its longitudinal axis covers a length from up to 1 mm to 5 cm, preferably from 0.25 cm to 3 cm, more preferably from 0.5 cm to 2 cm.

7. Piston pump system according to one of claims 1 to 6, **characterised in that** the O-ring seal (3) consists of silicone.

8. Piston pump system according to one of claims 1 to 7, **characterised in that** the piston (1) is a hollow piston in which the liquid-conveying connection with a valve (7) which connects the pumping chamber (5) to a storage vessel (11) is integrated.

9. Piston pump system according to one of claims 1 to 8, **characterised in that** the movement of the piston (1) is mechanically controlled.

10. Piston pump system according to claim 9, **characterised in that** the piston (1) is moved by means of a helical spring.

11. Piston pump system according to one of claims 1 to 8, **characterised in that** the movement of the piston is electronically controlled.

12. Piston pump system according to claim 11, **characterised in that** the piston is controlled by means of a microchip (18).

13. Piston pump system according to claim 11 or 12, **characterised in that** the piston is moved by means of a piezoelectric element.

14. Piston pump system according to one of the preceding claims, **characterised in that** the device for delivering the liquid is at least one nozzle, at least one micro-pin or at least one microcutter along which the liquid is guided, at least cannulas and/or at least one outlet.

15. Piston pump system according to one of the preceding claims, **characterised in that** the cord thickness of the O-ring (3) is from 0.3 to 3 mm, preferably 0.5 to 2 mm, more preferably 0.75 to 1.5 mm.

16. Medical device for delivering pharmaceutical liquids comprising a piston pump system according to one of claims 1 to 15.

17. Medical device according to claim 16, **characterised in that** the medical device is a transdermal therapeutic system which comprises in addition to the piston pump system a storage vessel consisting of at least one movable element or having a venting opening and at least one micro-pin or microcutter.

18. Medical device according to claim 16, **characterised in that** the medical device is a nebuliser for liquids, a nasal spray, an eye spray, an inhaler, preferably a propellant-free inhaler, a needleless injector or an infusion device.

## Revendications

1. Système de pompe à piston servant à doser des liquides pharmaceutiques comprenant un volume de pompe allant de 1 microlitre à 1 ml, constitué d'un piston (1) guidé dans un tube conducteur (2), lequel piston peut exécuter un mouvement de levage le long de son axe longitudinal et qui débouche dans une chambre de pompage (5), la chambre de pompage (5) étant reliée à un réservoir (11) par l'intermédiaire d'un raccordement conducteur de liquide doté d'une soupape (7), et un raccordement conducteur de liquide menant depuis la chambre de pompage (5) vers un dispositif (8) servant à distribuer le liquide, **caractérisé en ce que** le tube conducteur (2) comprend une rainure (4) dans laquelle est disposé un joint d'étanchéité torique (3), qui étanchéifie le piston (1), le joint d'étanchéité présentant un coefficient de perméation de gaz allant de 100 à 500 N*cm³*mm/(m²*h*bar) pour de l'azote (N₂), une compression radiale inférieure à 30 %, et le joint d'étanchéité (3) remplissant la rainure (4) à un niveau de remplissage supérieur à 90 %, et le piston (1) présentant une section transversale dont la longueur est comprise entre 0,75 et 2,25 mm.

2. Système de pompe à piston selon la revendication 1, **caractérisé en ce que** le niveau de remplissage de la rainure est supérieur à 95 %.

3. Système de pompe à piston selon la revendication 1 ou 2, **caractérisé en ce que** la soupape est une soupape anti-retour (7).

4. Système de pompe à piston selon l'une quelconque des revendications 1 à 3, **caractérisé en ce qu'**une soupape anti-retour (9) est réalisée dans le raccordement menant vers un dispositif servant à distribuer le liquide.

5. Système de pompe à piston selon l'une quelconque des revendications 1 à 4, **caractérisé en ce que** le piston (1) présente une longueur allant de 5 mm à 10 cm, de préférence allant de 1 cm à 7,5 cm.

6. Système de pompe à piston selon l'une quelconque des revendications 1 à 5, **caractérisé en ce que** le mouvement de levage du piston (1) couvre le long de l'axe longitudinal de ce dernier une longueur allant de 1 mm à 5 cm, de préférence allant de 0,25 cm à 3 cm, idéalement allant de 0,5 cm à 2 cm.

7. Système de pompe à piston selon l'une quelconque des revendications 1 à 6, **caractérisé en ce que** le joint d'étanchéité torique (3) est constitué de silicone.

8. Système de pompe à piston selon l'une quelconque des revendications 1 à 7, **caractérisé en ce que** le piston (1) est un piston creux, dans lequel est intégré le raccordement conducteur de liquide doté d'une soupape (7), lequel raccordement relie la chambre de pompage (5) à un réservoir (11).

9. Système de pompe à piston selon l'une quelconque des revendications 1 à 8, **caractérisé en ce que** le mouvement du piston (1) est commandé de manière mécanique.

10. Système de pompe à piston selon la revendication 9, **caractérisé en ce que** le piston (1) est déplacé par l'intermédiaire d'un ressort cylindrique.

11. Système de pompe à piston selon l'une quelconque des revendications 1 à 8, **caractérisé en ce que** le mouvement du piston est commandé de manière électronique.

12. Système de pompe à piston selon la revendication 11, **caractérisé en ce que** le piston est commandé au moyen d'une micropuce (18).

13. Système de pompe à piston selon la revendication 11 ou 12, **caractérisé en ce que** le piston est déplacé au moyen d'un élément piézoélectrique.

14. Système de pompe à piston selon l'une quelconque des revendications précédentes, **caractérisé en ce que** le dispositif servant à distribuer le liquide est au moins une buse, au moins une micropointe, au moins une microlame, le long de laquelle le liquide est guidé, au moins des canules et/ou au moins une sortie.

15. Système de pompe à piston selon l'une quelconque des revendications précédentes, **caractérisé en ce que** la solidité des cordons du joint torique (3) est comprise entre 0,3 et 3 mm, de préférence entre 0,5 et 2 mm, idéalement entre 0,75 et 1,5 mm.

16. Appareil médical servant à distribuer des liquides pharmaceutiques contenant un système de pompe à piston selon l'une quelconque des revendications 1 à 15.

17. Appareil médical selon la revendication 16, **caractérisé en ce que** ledit appareil médical est un système thérapeutique transdermique, lequel présente outre le système de pompe à piston, un réservoir constitué au moins d'un élément mobile ou doté d'une ouverture d'aération et au moins une micropointe ou une microlame.

18. Appareil médical selon la revendication 16, **caractérisé en ce que** l'appareil médical est un pulvérisateur pour liquides, un spray nasal, un spray oculaire, un inhalateur, de préférence un inhalateur exempt de gaz propulseur, un injecteur sans aiguille ou un appareil de perfusion.
